# EUROPÄISCHE PATENTSCHRIFT

(11) **EP 2 410 321 B1**
(45) Veröffentlichungstag und Bekanntmachung des Hinweises auf die Patenterteilung: **08.03.2017**
(21) Anmeldenummer: 10169615.1
(22) Anmeldetag: 15.07.2010
(51) Int. Cl.: G01N 27/28, G01N 27/333, G01N 33/18

(54) **Wasseranalyse-Sensorelektrode**
Water analysis sensor electrode
Electrode de capteur pour l'analyse d'eau

(43) Veröffentlichungstag der Anmeldung: 25.01.2012
(73) Patentinhaber: Hach Lange GmbH, 14163 Berlin (DE)
(72) Erfinder: Leyer, Axel, 41199 Mönchengladbach (DE); Heidemanns, Lothar, 41352, Korschenbroich (DE); Jonak, Andreas, 40667, Meerbusch (DE); Dr. Hahn, Markus, 47906 Kempen (DE); Kussmann, Michael, 40476, Düsseldorf (DE); Rudde, Heinz, 41836, Hückelhoven (DE); Rieger, Claudia, 40227 Düsseldorf (DE); Stellmach-Hanulok, Aurelia, 42489 Wülfrath (DE); Golitz, Andreas, 47445, Moers (DE)
(74) Vertreter: ter Smitten, Hans

(56) Entgegenhaltungen:
- DE-A1-102008 055 084
- DE-U1- 8 912 731
- DE-U1- 29 709 141
- US-A- 4 838 999

## Beschreibung

Die vorliegende Erfindung betrifft eine Wasseranalyse-Sensorelektrode zur Bestimmung eines Analyten in Wasser.

Die Sensorelektrode weist ein geschlossenes Sensorelektroden-Gehäuse mit einer Elektrolytlösung, beispielsweise mit einer pH-Pufferlösung, eine in der Elektrolytlösung angeordnete Messelektrode und eine im Sensorelektroden-Gehäuse eingeschlossene Gasblase auf. Das untere distale Ende des Sensorelektroden-Gehäuses ist mit einer ionenselektiven Sensorelektroden-Membran verschlossen. Die Gasblase ist erforderlich, um die temperaturabhängige Volumenänderung der Pufferlösung auszugleichen.

Derartige Sensorelektroden sind beispielsweise aus DE 10 2008 055 084 A1 bekannt. Die hierin offenbarte Sensorelektrode weist einen Innendurchmesser auf, der kleiner 15,0 mm ist. Wird eine Sensorelektrode mit einem derartig schmalen Durchmesser, beispielweise während des Transports auf den Kopf gedreht, so wandert die im Sensorelektroden-Gehäuses eingeschlossene Gasblase entgegen der Schwerkraft in den Bereich zwischen der Sensorelektroden-Membran und der Messelektrode, wo sie sich festsetzt. Wird die Sensorelektrode zur Montage um 180° wieder zurück gedreht, so verbleibt die Gasblase durch das Phänomen der Oberflächenspannung in diesem Bereich. Dieses Festsitzen der Gasblase führt dazu, dass die elektrische Verbindung zwischen der Sensorelektroden-Membran und der Messelektrode unterbrochen ist. Das Problem des Festsitzens der Gasblase wird üblicherweise dadurch überwunden, dass die Sensorelektrode vorsichtig angeschlagen wird, bis sich die Gasblase wieder freisetzt und entgegen der Schwerkraft nach oben steigt. Eine optische Kontrolle ist in der Regel nicht möglich, da das Gehäuse-Innere nicht sichtbar ist.

Aufgabe der Erfindung ist es demgegenüber, eine Wasseranalyse-Sensorelektrode zu schaffen, bei der ein Festsitzen der Gasblase im Inneren eines flüssigkeitsgefüllten Sensorelektroden-Gehäuses wirksam vermieden wird.

Diese Aufgabe wird erfindungsgemäß gelöst mit einer Wasseranalyse-Sensorelektrode mit den Merkmalen des Patentanspruchs 1.

Die Wasseranalyse-Sensorelektrode weist im Innern des Sensorelektroden-Gehäuses einen durchgehenden offenen Kapillarkanal auf, der sich über die axiale Länge des Sensorelektroden-Gehäuses an der Innenwand erstreckt. Der Kapillarkanal kann beispielsweise durch eine Kapillarnut in der Innenwand des Sensorelektroden-Gehäuses gebildet werden. Im Bereich der Kapillarnut ist der Effekt der Oberflächenspannung derart geschwächt, dass die Schwerkraft in der Regel ausreicht, die Elektrolytlösung unter eine untenliegende Gasblase fließen zu lassen. Bei Anwesenheit einer Gasblase im unteren Bereich der Sensorelektrode, d.h. im Bereich zwischen Sensorelektroden-Membran und der Messelektrode, wird die Gasblase infolge der Kapillarwirkung in dem Kapillarkanal von der im Sensorelektroden-Gehäuse befindlichen Elektrolytlösung unterspült. Die Elektrolytlösung fließt also durch den Kapillarkanal nach unten unter die Gasblase und zwingt diese, entgegen der Schwerkraft zu steigen, so dass die Gasblase aus dem besagten Bereich nach oben wandert.

Ein Stabelement ist in das Sensorelektroden-Gehäuse eingelegt, wobei das Stabelement im Innern des Sensorelektroden-Gehäuses an der Innenwand anliegt.

Die Dichte des für das Stabelement eingesetzten Materials ist größer als die Dichte der Elektrolytlösung, so dass das Stabelement auf dem Boden des Sensorelektroden-Gehäuses aufliegt und somit die Gasblase wirksam mit der Elektrolytlösung unterspült werden kann.

Besonders bevorzugt ist das Stabelement ein im Querschnitt kreisrundes, chemisch inertes Stabelement, das in das ebenfalls kreisrunde Sensorelektroden-Gehäuse eingelegt ist, wobei das Stabelement an der Gehäuse-Innenwand anliegt. Hierbei wird beim Anliegen des Stabelements an der Innenwand des Sensorelektroden-Gehäuses zu jeder Seite jeweils ein Kapillarkanal gebildet, der sich über die axiale Länge des an der Innenwand anliegenden Stabelements erstreckt.

Ferner kann im Innern des Sensorelektroden-Gehäuses, beispielsweise an der Unterseite des Verschlussdeckels ein Wandelement vorgesehen sein, das die Kippbewegung des Stabelements derart einschränkt, dass das Stabelement gegen ein Kippen sichert.

Das Stabelement kann im Innern des Sensorelektroden-Gehäuses ausschließlich durch Adhäsion an der Innenwand anliegen.

Das Stabelement muss jedoch keineswegs permanent an der Innenwand fixiert sein. Es kann sich von dieser Innenwand beispielsweise durch eine Schüttelbewegung vorübergehend lösen, kehrt aber selbsttätig durch die wirkenden Adhäsionskräfte an die Innenwand zurück.

Dadurch, dass das Stabelement chemisch inert ist, treten keine chemischen Wechselwirkungen mit der Elektrolytlösung auf, so dass die Messqualität der Sensorelektrode erhalten bleibt. Mögliche Materialien, aus denen das Stabelement bestehen kann, sind Glas, Metall, Keramiken oder Kunststoffe.

Vorzugsweise ist der Kapillarkanal im Querschnitt im Wesentlichen keilförmig ausgebildet. Alternativ kann der Kapillarkanal im Querschnitt im Wesentlichen eine kreisrunde Form aufweisen. Hierdurch wird im Innern des Sensorelektroden-Gehäuses ein offener Kapillarkanal ausgebildet, so dass der Effekt der Oberflächenspannung derart geschwächt wird, dass die Schwerkraft in der Regel ausreicht, um die Elektrolytlösung unter eine untenliegende Gasblase fließen zu lassen. Die Gasblase wird also wirksam von der Elektrolytlösung unterspült.

Vorzugsweise weist der Kapillarkanal an der Keilspitze einen Radius auf, der kleiner 1,0 mm ist. Die Keilspitze muss keineswegs absolut spitz ausgebildet sein. Vielmehr kann die Keilspitze, die von den sich schneidenden Kapillarkanalwänden gebildet wird, eine Rundung aufweisen, deren Radius jedoch kleiner 1,0 mm ist.

Gemäß einer bevorzugten Ausgestaltung wird zwischen den Kapillarkanalwänden ein Keilwinkel eingeschlossen, der kleiner ist als der durch das Gehäuse-Material, Elektrolytlösung und Gas definierte Kontaktwinkel. Als Kontaktwinkel wird der Winkel bezeichnet, den ein Tropfen einer bestimmten Flüssigkeit auf der planaren Oberfläche aus einem bestimmten Material zu dieser Oberfläche bildet.

Vorzugsweise ist das Verhältnis der Durchmesser von dem im Wesentlichen runden Stabelement zu dem im Wesentlichen kreisrunden Sensorelektroden-Gehäuse kleiner als 20. Hierdurch wird sichergestellt, dass der zwischen Stabelement und Innenwand gebildete Kapillarkanal einen ausrelchend kleinen Keilwinkel und damit die erforderliche Kapillarwirkung aufweist.

Vorzugsweise ist die axiale Länge des Stabelements mindestens 3,0 mm, besonders bevorzugt jedoch 5,0 mm größer als die Höhe bzw. der Pegel der Elektrolytlösung im Sensorelektroden-Gehäuse. Alternativ kann die axiale Länge des Stabelements mindestens 3 %, besonders bevorzugt 5 % größer sein als die Höhe bzw. der Pegel der Elektrolytlösung im Sensorelektroden-Gehäuse. Hierbei wird sichergestellt, dass das Stabelement in der Diagonalstellung dennoch in die Gasblase hineinragt, so dass die wirkenden Adhäsionskräfte ausreichend sind, um das Stabelement an die Innenwand zu ziehen. Zur Unterstützung dieser Bewegung kann im Bodenbereich des Sensorelektroden-Gehäuses ein Positionierungskegel vorgesehen sein, der das untere Ende des Stabelements, aufgrund der Schwerkraft, stets nach außen zur Innenwand schiebt.

Alternativ kann die axiale Länge des Stabelements mindestens 7/10 und maximal 98/100 der axialen Innenlänge des Sensorelektroden-Gehäuses sein. Das Stabelement ist derart im Innern des Sensorelektroden-Gehäuses angeordnet, dass zumindest ein Teil des Stabelements stets in die Gasblase hineinragt. Hierdurch wird gewährleistet, dass die Gasblase stets unterspült werden kann.

Vorzugsweise weist das Sensorelektroden-Gehäuse einen Innendurchmesser von weniger als 15,0 mm auf. Der Effekt des Festsitzens der Gasblase ist umso stärker, je kleiner der Innendurchmesser des Sensorelektroden-Gehäuses bei gleichbleibenden Gasblasenvolumen ist. Bei größeren Innendurchmessern ist ein Kapillarkanal daher entbehrlich.

Nachfolgend wird ein Ausführungsbeispiel der erfindungsgemäßen Sensorelektrode anhand der Zeichnung näher erläutert.

Es zeigen:
Figur 1 einen Längsschnitt der erfindungsgemäßen Sensorelektrode,
Figur 2 einen Querschnitt der Sensorelektrode der Figur 1, und
Figur 3 einen vergrößerten Ausschnitt aus der Querschnittsdarstellung der Figur 2.

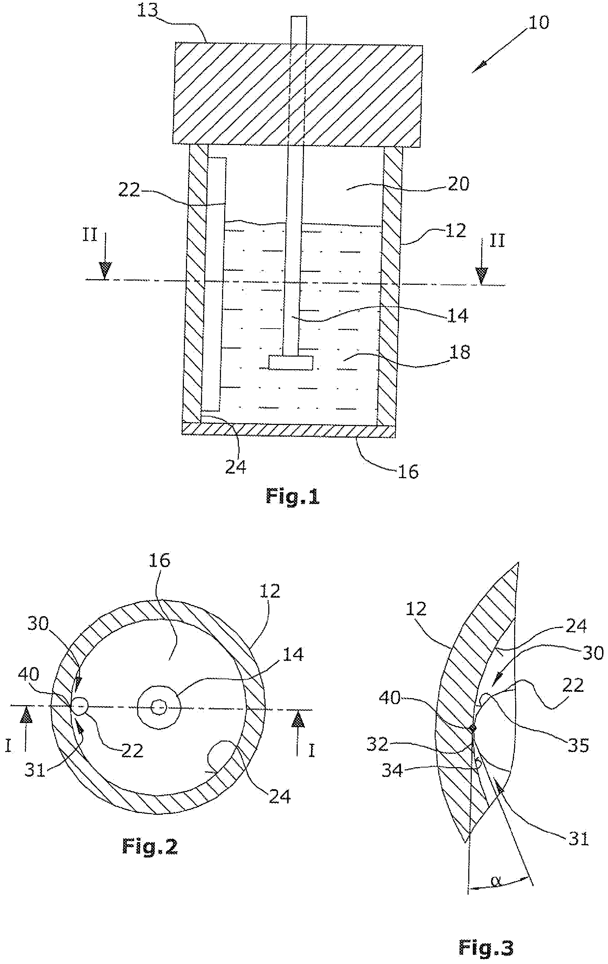

In Figur 1 ist eine Wasseranalyse-Sensorelektrode 10 zur Bestimmung eines Analyten in Wasser gezeigt. Die Sensorelektrode 10 besteht aus einem geschlossenen kreisrunden Sensorelektroden-Gehäuse 12 mit einem Innendurchmesser von ca. 6,0 mm, einer im Innern des Sensorelektroden-Gehäuses 12 auf der Gehäuse-Axialen angeordneten Messelektrode 14 in Form eines Ag/AgCl-Drahts und einer am unteren distalen Ende angeordneten ionenselektiven Sensorelektroden-Membran 16. Am entgegengesetzten Ende der Membran 16 ist das Sensorelektroden-Gehäuse 12 mit einem Verschlussdeckel 13 gasdicht verschlossen.

Das Sensorelektroden-Gehäuse 12 ist mit einer Elektrolytlösung 18 gefüllt und weist ferner eine eingeschlossene Gasblase 20 auf, beispielsweise eine Luftblase. In das Innere des Sensorelektroden-Gehäuses 12 ist ein Stabelement 22 eingelegt, beispielsweise ein Glasstab mit einem Durchmesser von 1,0 mm, welches an einer Innenwand 24 des Sensorelektroden-Gehäuses 12 adhäsiv anliegt. Ein Teil des Stabelements 22 ragt in die Gasblase 20 hinein. Idealerweise ist das Verhältnis des Durchmessers des runden Stabelements 22 zum Innendurchmesser des kreisrunden Sensorelektroden-Gehäuses 12 zueinander kleiner als zwanzig.

In Figur 2 ist eine Querschnittsdarstellung, in Figur 3 ein vergrößerter Ausschnitt der Querschnittsdarstellung der Sensorelektrode 10 aus Figur 1 gezeigt. Hierbei ist zu erkennen, dass beidseitig einer Berührungslinie 40 zwischen dem Stabelement 22 und der Innenwand 24 jeweils ein tangential offener und im Wesentlichen keilförmig ausgebildeter Kapillarkanal 30, 31 gebildet ist. Diese Kapillarkanäle 30, 31 erstrecken sich jeweils über die gesamte axiale Länge des Stabelements 22. Die von der Innenwand 24 gebildete Kapillarkanalwand 34 und die von dem Stabelement 22 gebildete Kapillarkanalwand 35 schließen einen Keilwinkel *α* ein, wobei der Keilwinkel *α* kleiner ist, als der Kontaktwinkel des Sensorelektroden-Gehäusematerials.

## Patentansprüche

1. Wasseranalyse-Sensorelektrode (10) zur Bestimmung eines Analyten in Wasser, bestehend aus:
einem geschlossenen Sensorelektroden-Gehäuse (12) mit einer Elektrolytlösung (18), einer Messelektrode (14) und einer im Sensorelektroden-Gehäuse (12) eingeschlossenen Gasblase (20), und einer am unteren distalen Ende des Sensorelektroden-Gehäuses (12) angeordneten ionenselektiven Sensorelektroden-Membran (16),
**dadurch gekennzeichnet,**
**dass** ein Stabelement (22) in das Sensorelektroden-Gehäuse (12) eingelegt ist, wobei das Stabelement (22) im Inneren des Sensorelektroden-Gehäuses (12) an der Innenwand (24) derart anliegt,
**dass** sich über die Länge des Sensorelektroden-Gehäuses (12) an der Innenwand (24) ein durchgehender offener Kapillarkanal (30) erstreckt, und
**dass** die Dichte des Stabelement-Materials größer als die Dichte der Elektrolytlösung ist, so dass das Stabelement auf dem Boden des Sensorelektroden-Gehäuses aufliegt.

2. Wasseranalyse-Sensorelektrode (10) nach Anspruch 1, wobei der Kapillarkanal (30) im Querschnitt im Wesentlichen keilförmig ausgebildet ist.

3. Wasseranalyse-Sensorelektrode (10) nach Anspruch 1 oder 2, wobei der Kapillarkanal (30) an der Keilspitze (32) einen Radius kleiner 1,0 mm aufweist.

4. Wasseranalyse-Sensorelektrode (10) nach einem der vorangegangenen Ansprüche, wobei die Kapillarkanalwände (34, 35) einen Keilwinkel (*α*) einschließen, der kleiner ist als der durch das Gehäuse-Material, die Elektrolytlösung (18) und Gas definierte Kontaktwinkel.

5. Wasseranalyse-Sensorelektrode (10) nach einem der vorangegangenen Ansprüche, wobei das Stabelement (22) im Inneren des Sensorelektroden-Gehäuses (12) durch Adhäsion an der Innenwand (24) anliegt.

6. Wasseranalyse-Sensorelektrode (10) nach einem der vorangegangenen Ansprüche, wobei das Verhältnis der Durchmesser von dem runden Stabelement (22) zu dem kreisrunden Sensorelektroden-Gehäuse (12) kleiner als 20 ist.

7. Wasseranalyse-Sensorelektrode (10) nach einem der vorangegangenen Ansprüche, wobei die axiale Länge des Stabelements (22) mindestens 3,0 mm, vorzugsweise 5,0 mm größer ist als die Höhe der Elektrolytlösung (18) im Sensorelektroden-Gehäuse (12).

8. Wasseranalyse-Sensorelektrode (10) nach einem der Ansprüche 1 - 6, wobei die axiale Länge des Stabelements (22) mindestens 3 %, vorzugsweise 5 % länger ist als die Höhe der Elektrolytlösung (18) im Sensorelektroden-Gehäuse (12).

9. Wasseranalyse-Sensorelektrode (10) nach einem der Ansprüche 1 - 6, wobei die axiale Länge des Stabelements (22) mindestens 7/10 und maximal 98/100 der axialen Innenlänge des Sensorelektroden-Gehäuses (12) beträgt.

10. Wasseranalyse-Sensorelektrode (10) nach einem der vorangegangenen Ansprüche, wobei das Sensorelektroden-Gehäuse (12) einen Innendurchmesser von kleiner 15,0 mm aufweist.

## Claims

1. A water analysis sensor electrode (10) for determining an analyte in water, comprising:
a closed sensor electrode housing (12) with an electrolyte solution (18), a measuring electrode (14) an a gas bubble (20) enclosed in the sensor electrode housing (12), and
an ion-selective sensor electrode diaphragm (16) arranged at the lower distal end of the sensor electrode housing (12),
**characterized in that**
a rod element (22) is placed in the sensor electrode housing (12) so that a continuous open capillary channel (30) extends over the length of the sensor electrode housing (12) between the rod element (22) and the inner wall (24), and the density of the material of the rod element (22) is higher than the density of the electrolyte solution (18) so that the rod element rests on the bottom of the sensor electrode housing.

2. A water analysis sensor electrode (10) of claim 1, wherein the capillary channel is wedge-like in cross section.

3. A water analysis sensor electrode (10) of claim 1 or 2, wherein the radius at the wedge tip is less than 1,0 mm.

4. The water analysis sensor electrode (10) of one of the preceding claims,
wherein the capillary channel walls (34, 35) include a wedge angle (α) that is smaller than the contact angle defined by the housing material, the electrolyte solution (18) and the gas.

5. The water analysis sensor electrode (10) of claim 1, wherein the rod element (22) abuts by adhesion on the inner wall (34) inside the sensor electrode housing (12).

6. The water analysis sensor electrode (10) of one of the preceding claims,
wherein the ratio of the diameters of the round rod element (22) to the circular sensor electrode housing (12) is less than 20.

7. The water analysis sensor electrode (10) of one of the preceding claims,
wherein the axial length of the rod element (22) is at least 3.0 mm, preferably 5.0 mm larger than the level of the electrolyte solution (18) in the sensor electrode housing (12).

8. The water analysis sensor electrode (10) of one of claims 1-6, wherein the axial length of the rod element (22) is at least 3%, preferably 5% longer than the level of the electrolyte solution (18) in the sensor electrode housing (12).

9. The water analysis sensor electrode (10) of one of claims 1-6, wherein the axial length of the rod element (22) is at least 7/10 and at most 98/100 of the axial inner length o he sensor electrode housing (12).

10. The water analysis sensor electrode (10) of one of the preceding claims,
wherein the sensor electrode housing (12) has an inner diameter smaller than 15.0 mm.

## Revendications

1. Electrode de capteur pour l'analyse d'eau (10) pour la détermination d'un analyte dans l'eau, constitué par:
un carter d'électrode de capteur (12) fermé avec une solution d'électrolyte (18), une électrode de détection (14) et une bulle de gaz (20) renfermée dans le carter d'électrode de capteur (12), et
une membrane d'électrode de capteur (16) sélectivement sensible aux ions, qui est disposée à l'extrémité basse du carter d'électrode de capteur (12),
**caractérisée en ce**
**qu'**un élément de tige (22) est placé dans le carter d'électrode de capteur (12), ledit élément de tige (22) reste contre la paroi intérieure (24) dans l'intérieur du carter d'électrode de capteur (12) de sorte
**qu'**un canal capillaire (30) continu et ouvert s'étend sur la paroi intérieure (24) selon la longueur du carter d'électrode de capteur (12), et
**que** la densité du matériel dudit élément de tige est supérieure à la densité de la solution d'électrolyte, de sorte que ledit élément de tige reste sur le fond du carter d'électrode de capteur.

2. Electrode de capteur pour l'analyse d'eau (10) selon la revendication 1,
dans laquelle, en coupe transversale, le canal capillaire (30) est agencé sensiblement en forme de coin.

3. Electrode de capteur pour l'analyse d'eau (10) selon la revendication 1 ou 2, dans laquelle, à la pointe du coin (32), le canal capillaire (30) a un radius inférieur à 1,0 mm.

4. Electrode de capteur pour l'analyse d'eau (10) selon l'une quelconque des revendications précédentes, dans laquelle les parois du canal capillaire (34, 35) forment un angle de coin (α) inférieur à l'angle de contact défini par le matériel du carter, la solution d'électrolyte (18) et le gaz.

5. Electrode de capteur pour l'analyse d'eau (10) selon l'une quelconque des revendications précédentes, dans laquelle ledit élément de tige (22) reste contre la paroi intérieure (24) dans l'intérieur du carter d'électrode de capteur (12) par adhésion.

6. Electrode de capteur pour l'analyse d'eau (10) selon l'une quelconque des revendications précédentes, dans laquelle le rapport des diamètres entre ledit élément de tige (22) rond et le carter d'électrode de capteur (12) circulaire est inférieur à 20.

7. Electrode de capteur pour l'analyse d'eau (10) selon l'une quelconque des revendications précédentes, dans laquelle la longueur axiale dudit élément de tige (22) est au moins 3,0 mm, de préférence 5,0 mm plus grande que le niveau de la solution d'électrolyte (18) dans le carter d'électrode de capteur (12).

8. Electrode de capteur pour l'analyse d'eau (10) selon l'une quelconque des revendications 1 - 6, dans laquelle la longueur axiale dudit élément de tige (22) est au moins 3%, de préférence 5% plus longue que le niveau de la solution d'électrolyte (18) dans le carter d'électrode de capteur (12).

9. Electrode de capteur pour l'analyse d'eau (10) selon l'une quelconque des revendications 1 - 6, dans laquelle la longueur axiale dudit élément de tige (22) est au moins 7/10 et au maximum 98/100 de la longueur intérieure axiale du carter d'électrode de capteur (12).

10. Electrode de capteur pour l'analyse d'eau (10) selon l'une quelconque des revendications précédentes, dans laquelle le carter d'électrode de capteur (12) a un diamètre intérieur inférieur à 15,0 mm.
